## Europäisches Patentamt

## European Patent Office

## Office européen des brevets

(11) Veröffentlichungsnummer: **0 223 218 A2**

(12) # EUROPÄISCHE PATENTANMELDUNG

(21) Anmeldenummer: 86115890.5

(51) Int. Cl.⁴: **A 61 K 39/00, A 61 K 35/12**

(22) Anmeldetag: 15.11.86

(30) Priorität: 22.11.85 DE 3541303

(71) Anmelder: **Stang-Voss, Christiane, Prof. Dr., Hauptstrasse 533, D-5330 Königswinter (DE)**

(43) Veröffentlichungstag der Anmeldung: 27.05.87 **Patentblatt 87/22**

(72) Erfinder: **Stang-Voss, Christiane, Prof. Dr., Hauptstrasse 533, D-5330 Königswinter (DE)**
Erfinder: **Appell, Hans-Joachim, Dr., Kölner Weg 38, D-5000 Köln 40 (DE)**

(84) Benannte Vertragsstaaten: **AT BE CH DE ES FR GB GR IT LI LU NL SE**

(74) Vertreter: **Werner, Hans-Karsten, Dr. et al, Deichmannhaus am Hauptbahnhof, D-5000 Köln 1 (DE)**

(54) Konditionierte Makrophagen und Verfahren zu ihrer Herstellung.

(57) In vitro-immunrelevant informierte Makrophagen und Verfahren zu deren Herstellung, wobei frisch gewonnene Makrophagen in einem vorzugsweise serumfreien Medium kultiviert und dabei von Lymphozyten und anderen Verunreinigungen gereinigt, die so gewonnenen adhärenten Makrophagen mit Antigenen versetzt und zur Reaktion gebracht und die reaktiven adhärenten Makrophagen zur Verwendung für die Injektion in einen Empfänger abgelöst werden.

## Konditionierte Makrophagen und Verfahren zu ihrer Herstellung

Die vorliegende Erfindung betrifft konditionierte Makrophagen sowie ein Verfahren zu deren In-vitro-Herstellung.

Es ist bekannt, daß maligne Neoplasmen Immunreaktionen hervorrufen können. In einigen Fällen kann dies zu einer Resistenz gegen wachsende Tumorgewebe führen. So können Mäuse, die geringe Mengen von Tumorzellen erhalten haben, größere Mengen von später gebildeten neoplastischen Zellen eliminieren. Dasselbe kann auch mit anderen syngenen Tieren durchgeführt werden, wenn man Lymphozyten von mit Tumoren befallenen Mäusen verwendet (vgl. Nakayama M., Sando F., Miyake T., Fuyama S., Arai S. and Kobayachi H.: Nonspecific inhibition of tumor growth by allosensitized lymphocytes; Participation of polynuclear leukocytes; J. Immunol 120: 619-628, 1978; and van Loveren H., Snoek M. and den Otter W.: Host macrophages are involved in systemic adoptive immunity against tumors; Experientia 38: 488-489, 1982). Voraussetzung für diese Immunreaktion ist allerdings ein intaktes Makrophagensystem. Eine tumorspezifische Immunität kann ferner durch die Behandlung mit Glykolipidextrakten (vgl. Ansel S. and Blangy D.: In vivo induction of tumor-specific immunity by glycolipid extracts of SV 40-transformed cells; Int. J. Cancer 34: 555-559, 1984) oder mit Lipopolysaccharid aktivierten Makrophagen (vgl. Cameron D.J.: In vivo macrophage-mediated tumor cytotoxicity in the mouse; Immunol Lett 4: 321-325, 1982) induziert werden.

Entsprechende Behandlungen sind beim Menschen nur erfolgreich, wenn mit eineiigen Zwillingen gearbeitet wird. Allogene Lymphozyten oder Makrophagen würden nämlich sofort eliminiert werden oder sie würden eine Abwehrreaktion des behandelten Organismus hervorrufen. Eine tumorspezifische Immunität läßt sich beim Menschen also nur erreichen, wenn ein syngenes System vorliegt. Dies muß als Träger dienen, welcher die Tumor-Antigen-Information in sich trägt, ohne selbst Tumorwachstum zu induzieren (vgl. Ng A K., Zhang Y H., Russo C. and Ferrone S.: Immunochemical and functional analysis of Ia-like antigens on human monocyte-macrophages with monoclonal antibodies; Cell Immunol 66: 78-87, 1982).

Bei allen höheren Säugern spielen die Makrophagen eine Schlüsselrolle in deren Immunsystem. Durch sie werden Antigene internalisiert. Nach einem sogenannten Processing erfolgt die Präsentation gegenüber den reaktiven T-Zellen und spezifischen B-Zellen. Diese Präsentation ist beschränkt auf den Haupt-Histokompatibilitätskomplex (MHC) auf der Oberfläche der Zellmembran (vgl. Mercurio A.M., Schwarting G.A. and Robbins P.W.: Glycolipids of the mouse peritoneal macrophage; J. exp Med 160: 1114-1125, 1984; and Unanue E.R.: Antigen-presenting function of the macrophage; Ann Rev. Immunol 2: 395-428, 1984). In ähnlicher Weise starten die Makrophagen eine kaskadenähnliche Immunantwort im Immunsystem (vgl. Fischer H.G., Reske-Kunz A.B., Spaeth E., Kirchner H. and Rüde E.: Characerization of lymphokine-mediated activation of macrophages for antigen presentation; studies with long-term cultured bone marrow-derived macrophages and cloned T-cells; Immunbiol 168: 232-245, 1984).

Wenn es möglich wäre, eine Antigenpräsentation in vitro einzuleiten und diese so konditionierten Makrophagen in einen syngenen Wirtsorganismus zu übertragen, könnte der Organismus spezifisch immunisiert werden. Jedoch hängt die Präsentation von Antigenen von der Expression der Ia-Antigene auf der Oberfläche der Makrophagen ab.

Die vorliegende Erfindung betrifft demgemäß in vitro-immunrelevant informierte Makrophagen. Darüber hinaus hat sich die Erfindung die Aufgabe gestellt, ein Verfahren zur In-vitro-Herstellung von immunrelevant informierten Makrophagen zur Verfügung zu stellen.

Diese Aufgabe wird dadurch gelöst, daß

a)  frisch gewonnene Makrophagen in einem vorzugsweise serumfreien Medium kultiviert und dabei von Lymphozyten und anderen Verunreinigungen gereinigt,

b)  die so gewonnenen adhärenten Makrophagen mit Antigenen versetzt und zur Reaktion gebracht und

c)  die reaktiven adhärenten Makrophagen abgelöst werden.

Außerdem ist Gegenstand der vorliegenden Erfindung die Verwendung von in vitro-immunrelevant informierten Makrophagen zur Herstellung einer injizierbaren immunisierenden Suspension, die gegebenenfalls durch Schockgefrieren, durch Formaldehydbehandlung oder andere Methoden stabilisiert werden kann.

In dem erfindungsgemäßen Verfahren werden zunächst aus einem Säugetierorganismus Makrophagen gewonnen. Diese werden anschließend 3 bis 8 h in einem Medium kultiviert, das vorzugsweise frei von Serum ist. Bevorzugt wird eine Kultivierungszeit von 5 bis 6 h, insbesondere 6 h.

Die Makrophagen setzen sich an dem Boden des Kulturgefäßes fest, während die Lymphozyten sich an der Oberfläche der Nährlösung befinden. Im Laufe der Kultivierung werden die Lymphozyten regelmäßig aus dem Überstand entfernt.

Im Anschluß an die Kultivierung werden die nun in hochreiner Form vorliegenden Makrophagen mit Antigenen versetzt. Die Reaktionszeit beträgt 1 bis 3 h. Anschließend werden die so konditionierten Makrophagen abgelöst. Geeignete Lösungsmittel sind Trypsin und Ethylendiamintetraessigsäure (EDTA). Die erhaltenen Makrophagen werden zur Herstellung einer injizierbaren und immunisierenden Suspension verwendet. Vorzugsweise wird in einem geeigneten Nährmedium oder in NaCl suspendiert. Die so entstandene Suspension wird einem Säugetier injiziert. Versuche der Anmelderin haben ergeben, daß bei der Injektion in Mäuse kaum Tumorwachstum zu beobachten war im Vergleich zu den Tieren, die keine konditionierten Makrophagen erhalten hatten. Bei diesen Versuchen hat sich gezeigt, daß es wichtig ist, daß zwischen der Ablösung der Makrophagen und ihrer Injektion in den Wirtstierorganismus möglichst wenig Zeit vergeht, denn die Präsentation von Antigenen dauert nur kurze Zeit. Ebenso ist anscheinend das Processing der Antigene innerhalb einer Stunde abgeschlossen. Deshalb bleibt nur kurze Zeit, um die in vitro-modifizierten Makrophagen in einen Wirtstierorganismus zu transplantieren.

Das Prinzip des erfindungsgemäßen Verfahrens wird durch die Zeichnungen näher erläutert:

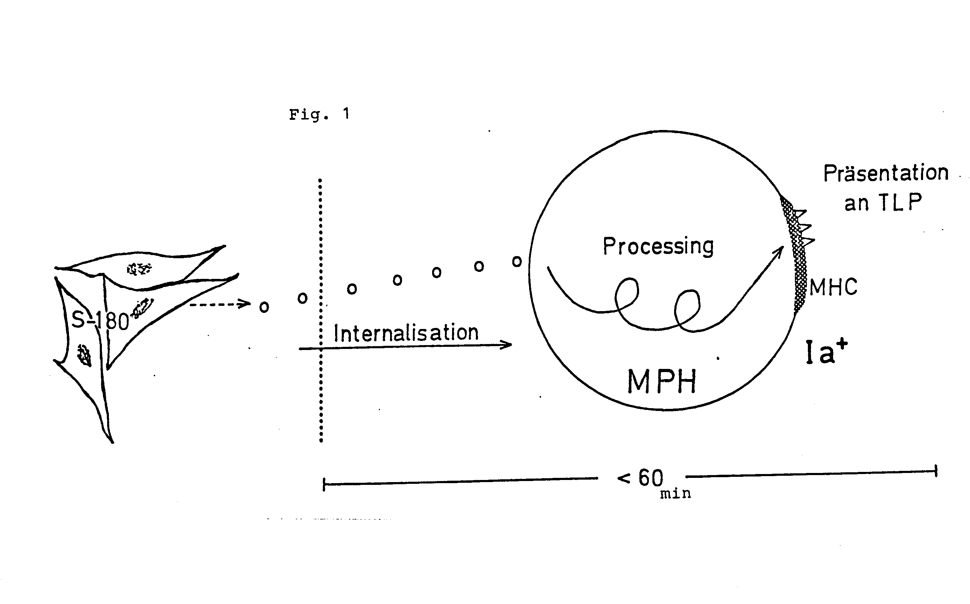

Fig. 1 zeigt das Prinzip der Internalisation eines Antigens durch einen Makrophagen (MPH) sowie das anschließende Processing und die Präsentation an einen T-Lymphozyten-Precursor (TL-P).

Fig. 2 zeigt den Ablauf von der Internalisation des Antigens durch Makrophagen bis zur Antikörper-Bildung.

Danach präsentiert ein Makrophage (MPH) das Antigen einem T-Lymphozyten-Precursor (TL-P). Über Helper-T-Lymphozyten (HTL) und B-Lymphozyten (B) kann es schließlich zur Antikörper-Bildung kommen. Über die Helper-T-Lymphozyten und die cytotoxischen T-Lymphozyten (CTL) kann es aber auch gleichzeitig zu einer Rückkoppelung zum Makrophagen kommen. Abgesehen von den durch die Antigen-Internalisation gebildeten spezifischen Makrophagen können so auch Makrophagen unspezifisch aktiviert werden, die die verschiedensten Substanzen bilden. Hierzu zählen zum Beispiel Prostaglandin (PgE$_2$), tumornekrotische Faktoren (TNF), Interferon $\alpha$, $\beta$ (IFN) und H$_2$O$_2$.

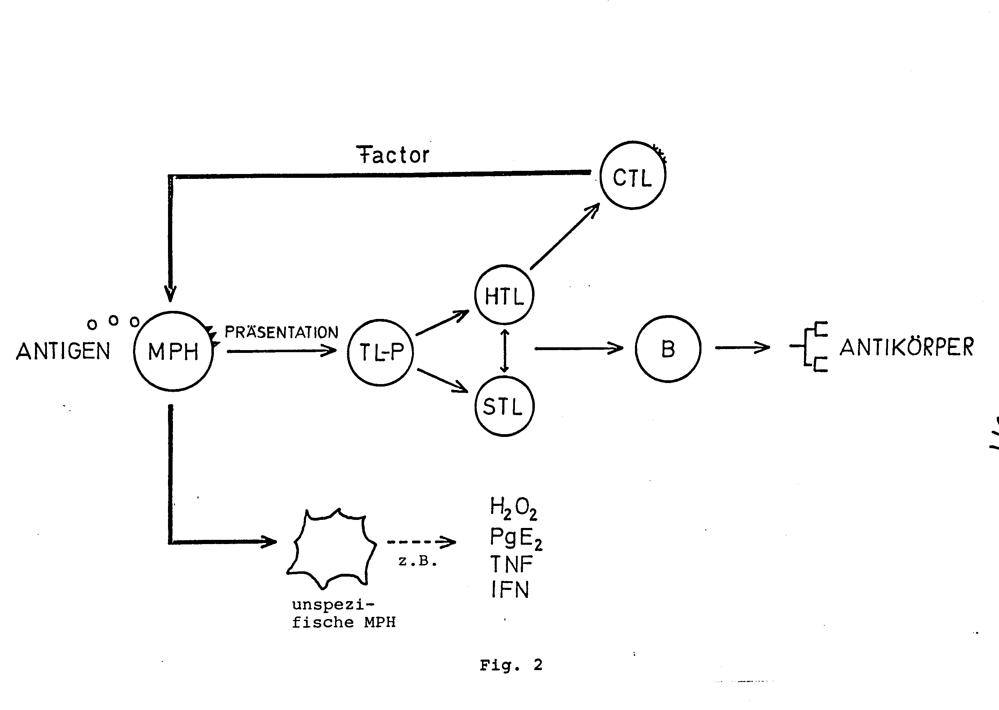

In den Wirtstieren werden die Antigene wahrscheinlich dem Helper-T-Lymphozyten-Precursor (HTLP) bei gleichzeitiger Abgabe von Interleukin I (Il 1) von den Makrophagen präsentiert (vgl. Fig. 2). Dies ist wichtig für eine Transformation des HTLP zum Helper-T-Lymphozyten (HTL), welcher Interleukin II (Il 2) produziert. Erst dann vermehren sich auch cytotoxische T-Lymphozyten (CTL).

Die Akkumulation von Interferon $\gamma$ (IFN$\gamma$) und dem Makrophagen-Cytotoxizität induzierenden Faktor 2 (MCIF2) sowie wahrscheinlich eine Antikörperproduktion durch das B-Lymphozytensystem (B) verbessern die tumorspezifische Cytotoxizität des gesamten Makrophagensystems des Organismus. Dies führt schließlich zu einer Eliminierung der künstlich zugesetzten Tumorzellen bzw. zur Verhinderung von Tumorwachstum. So ist von de Boer (vgl. de Boer R., Hogeweg P., Dullens H.F.J., de Weger R.A. and den Otter W.: Macrophage T-lymphocyte interactions in the anti-tumor immune response; a mathematical model., J. Immunol 134: 2748-2758, 1985) demonstriert worden, daß nur ein HTL benötigt wird, um 50000 Tumorzellen zu zerstören. Dies mag eine Erklärung dafür liefern, daß nur eine geringe Anzahl von konditionierten Makrophagen nötig ist, um eine spezifische Tumorimmunität zu erreichen.

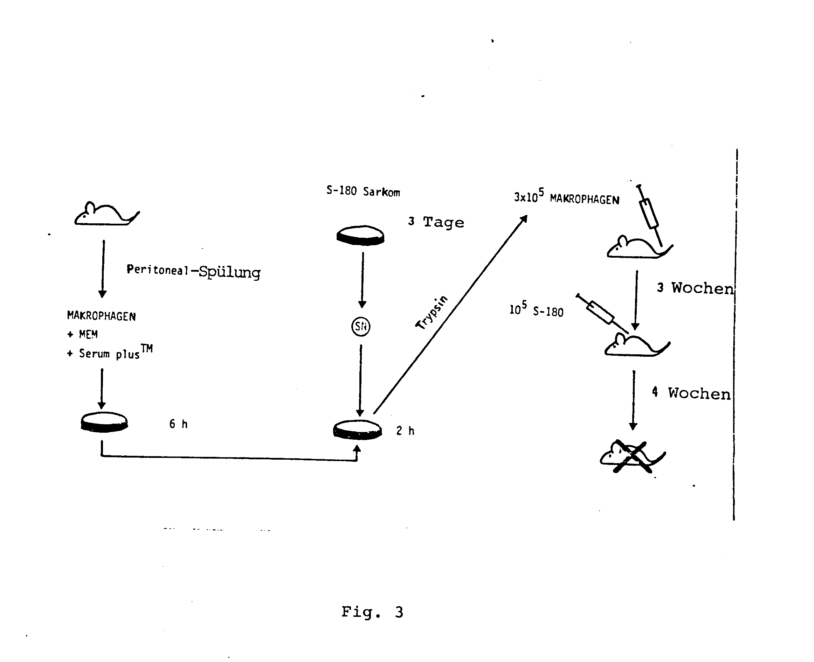

Fig. 3 zeigt den Gesamtablauf des erfindungsgemäßen Verfahrens sowie die Verwendung der erfindungsgemäßen Substanzen für die Injektion am Beispiel von Mäusen. Hiernach werden zunächst aus der Maus die Makrophagen gewonnen und 6 h kultiviert. Danach wird 2 h in dem Überstand einer S-180 Sarkom-Zellkultur konditioniert. Nach der anschließenden Ablösung der adhärenten Makrophagen mit Trypsin und deren Suspendierung wird in die Schwanzvene der Maus injiziert und das Wachstum eines drei Wochen später inokulierten Tumors bei den Mäusen beobachtet.

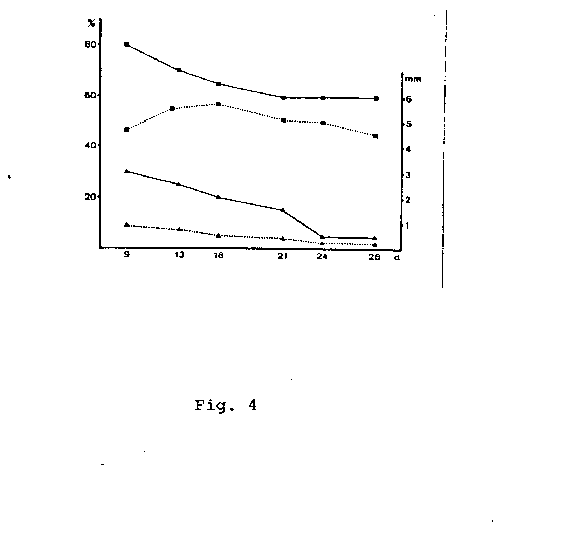

Fig. 4 zeigt schließlich die Ergebnisse des im nachfolgenden Beispiel geschilderten Versuchs:

## B e i s p i e l :

6 Wochen alte Mäuse (SPF-NMRI) wurden als Makrophagen-Spender und Tumorwirtstiere benutzt. Sie wurden unter Standardbedingungen gehalten (Tag/ Nacht-Zyklus 12 h, Temperatur 22 °C, relative Feuchtigkeit 55 %, Altromin-Standardfutter und Wasser nach Belieben). 20 Mäuse wurden für das Experiment benutzt; dieselbe Anzahl diente als Kontrollgruppe.

Für die Konditionierung der Makrophagen und ihrer Anwendung wurden S-180 Sarkom-Zellen verwendet. Nach der Inokulierung von $10^5$ Zellen kann dieser Tumor innerhalb von 2 Wochen bis zu nahezu 2 cm Durchmesser anwachsen. Die S-180-Zellen wurden in einem Standardmedium (Eagle's MEM = Minimum essential Medium) und 10 % Serum plus$^{TM}$ kultiviert.

Die Makrophagen wurden aus den Mäusen durch Peritoneal-Spülung mit MEM gewonnen. 90 % der gewonnenen Zellen sind gewöhnlich Makrophagen (vgl. Michna H.: Die Aktivität des Makrophagen-Systems des Menschen; Dissertation, Lübeck, 1984). Mindestens $3x10^6$ Zellen in 3 ml MEM mit 10 % Serum plus$^{TM}$ wurden in 21 cm² Falcon Petri-Schalen gegeben. Nach der Inkubation bei 37 °C für 6 h wurden 2 ml des Überstandes (SN) von einer 3 Tage alten S-180 Sarkom-Zellkultur zu den Makrophagen hinzugefügt und für weitere 2 h inkubiert (vgl. hierzu auch Fig. 3).

Die konditionierten Makrophagen wurden trypsiniert, mit MEM gespült und bei 600 upm für 10 min zentrifugiert. $3x10^5$ Makrophagen wurden in 0,3 ml MEM dispergiert und in die Schwanzvene der Maus injiziert. Hierbei wurde darauf geachtet, daß zwischen Trypsinierung und Injizierung der Makrophagen nicht mehr als 60 min vergingen.

Nach 3 Wochen wurden den mit konditionierten Makrophagen behandelten Tieren $10^5$ S-180 Sarkom-Zellen verabreicht, indem man unter der Nackenhaut inokulierte. Das Wachstum der S-180 Sarkom-Zellen wurde durch Betasten ermittelt und die mittleren Tumordurchmesser wurden kalibriert. Tumorfreie Mäuse, welche mit Makrophagen behandelt worden waren, wurden nach 9 Wochen wieder mit $10^5$ S-180 Sarkom-Zellen inokuliert.

Die Mäuse der Kontrollgruppe erhielten dieselbe Menge an S-180 Sarkom-Zellen. Sie waren jedoch nicht mit konditionierten Makrophagen vorbehandelt worden. Das Wachstum des Tumors wurde ebenso wie in der mit Makrophagen behandelten Gruppe ermittelt.

4 Wochen nach der ersten Inokulierung mit S-180 Sarkom-Zellen und 4 Wochen nach der zweiten Inokulierung wurden die Nackenregionen der Mäuse makroskopisch und histologisch untersucht, um festzustellen, ob die Tumorzellen eliminiert worden waren.

In der Kontrollgruppe (Fig. 4) hatten 80 % der Tiere ertastbare Tumoren mit einem mittleren Durchmesser von 4,6 mm (Bereich von 2 bis 20 mm) 9 Tage nach der Inokulierung mit S-180 Sarkom-Zellen entwickelt. Die Tumorgröße wuchs allmählich auf 5,6 mm nach 16 Tagen an und nahm nach 4 Wochen auf 4,5 mm ab. Am Ende des Untersuchungszeitraums (4 Wochen) hatten 50 % der mit Tumoren befallenen Tiere den Tumor wieder abgestoßen. In der mit Makrophagen behandelten Gruppe hatten nur 30 % der Mäuse nach 9 Tagen ein Tumorwachstum aufzuweisen, welches allerdings kaum zu ertasten war (durchschnittlicher Durchmesser von weniger als 1 mm). Aber auch diese kleinen Tumoren verschwanden nach 24 bis 28 Tagen mit einer Ausnahme.

Die tumorfreien Tiere (n=14) entwickelten auch nach der zweiten Behandlung mit S-180 Sarkom-Zellen kein Tumorwachstum.

Die Ergebnisse zeigen, daß nach der Applikation von konditionierten Makrophagen die Mäuse eine spezifische Tumorimmunität gegen S-180 Sarkom-Zellen aufweisen. Die Tatsache, daß 20 % der Kontrollgruppe tumorfrei geblieben sind, kann damit erklärt werden, daß diese Tiere selbst Antikörper gegen die Tumorzellen entwickelt haben. Die Tumoren, die sich in nur 30 % der mit Makrophagen behandelten Tiere entwickelt hatten, besaßen nur geringe Größe und waren nach kurzer Zeit völlig verschwunden. Für diesen Vorgang sind vermutlich zwei Gründe ausschlaggebend:

1. Die Zahl der Tiermakrophagen wurde durch die aktivierten Makrophagen des Donators vergrößert, so daß auf diese Weise die unspezifische Tumorcytotoxizität verbessert wurde.

2. Die Verabreichung von konditionierten Makrophagen hatte das Lymphozytensystem durch die Antigenpräsentation aktiviert, so daß tumorspezifische Antikörper produziert wurden.

Wahrscheinlich ist der zweite Punkt der wichtigere Wirkungsmechanismus. Vermutlich führt die Behandlung mit nach dem erfindungsgemäßen Verfahren hergestellten Makrophagen zu einem Langzeiteffekt aufgrund der Existenz von S-180-Antikörpern. Diese Behauptung wird dadurch belegt, daß eine zweite Inokulierung von S-180 Sarkom-Zellen nach 9 Wochen (gerechnet vom Ende des ersten Experiments an) zu keiner Entwicklung von Tumoren in der lebenden Zelle führte.

In Serien von Vorversuchen wurde herausgefunden, daß der Erfolg der Methode von der Zeit zwischen der Trypsinierung der adhärenten Makrophagen und ihrer Transplantation in das Versuchstier abhängt.

Es ist bekannt, daß menschliche Makrophagen leicht mit der sogenannten Cantharidin-Methode gewonnen werden können (vgl. Ng A K., Zhang Y H., Russo C. and Ferrone S.: Immunochemical and functional analysis of Ia-like antigens on human monocyte-macrophages with monoclonal antibodies; Cell Immunol 66: 78-87, 1982). Aus mit Cantharidin induzierten Hautblasen können $2,4 \times 10^6$ Makrophagen erhalten werden, welche zu 45 % Ia-positiv sind. Diese können dann in gleicher Weise wie oben in der Beschreibung und dem Beispiel geschildert, konditioniert werden. Auf diese Weise wird ein wirkungsvolles Mittel zur Bekämpfung von Tumorerkrankungen im Menschen zur Verfügung gestellt, dessen besonderer Vorteil in seiner Langzeitwirkung liegt. In gleicher Weise können diese Makrophagen mit anderen Antigenen informiert werden und zur Immunisierung verwendet werden. Vorzugsweise wird man Makrophagen des zu immunisierenden Patienten verwenden. Aufgrund der geringen Menge von konditionierten Makrophagen, die prinzipiell für die Erzielung einer Immunität notwendig ist, können möglicherweise auch Makrophagen anderer Herkunft als Antigen-Präsentatoren benutzt werden, ohne daß eine störende Abwehrreaktion des Patienten auftritt.

## Patentansprüche

1. In vitro-immunrelevant informierte Makrophagen.

2. Makrophagen gemäß Anspruch 1, dadurch gekennzeichnet, daß sie durch Oberflächen-Antigene informiert sind.

3. Makrophagen gemäß einem der Ansprüche 1 oder 2, dadurch gekennzeichnet, daß sie durch Tumor-Antigene informiert sind.

4. Verfahren zur In-vitro-Herstellung von immunrelevant informierten Makrophagen, dadurch gekennzeichnet, daß

   a) frisch gewonnene Makrophagen in einem vorzugsweise serumfreien Medium kultiviert und dabei von Lymphozyten und anderen Verunreinigungen gereinigt,

   b) die so gewonnenen adhärenten Makrophagen mit Antigenen versetzt und zur Reaktion gebracht und

   c) die reaktiven adhärenten Makrophagen abgelöst werden.

5. Verfahren nach Anspruch 4, dadurch gekennzeichnet, daß die Makrophagen 3 bis 8 h kultiviert werden.

6. Verfahren nach Anspruch 5, dadurch gekennzeichnet, daß die Makrophagen 5 bis 6 h kultiviert werden.

7. Verfahren nach einem der Ansprüche 4 bis 6, dadurch gekennzeichnet, daß die Makrophagen 1 bis 3 h mit den Antigenen zur Reaktion gebracht werden.

8. Verfahren nach Anspruch 7, dadurch gekennzeichnet, daß die Makrophagen 2 h reagieren.

9. Verfahren nach einem der Ansprüche 4 bis 8, dadurch gekennzeichnet, daß die Makrophagen mit Trypsin oder Ethylendiamintetraessigsäure (EDTA) abgelöst werden.

10. Verwendung von in vitro-immunrelevant informierten Makrophagen zur Herstellung einer injizierbaren immunisierenden Suspension.

11. Verwendung von in vitro-immunrelevant informierten Makorphagen nach Anspruch 10, dadurch gekennzeichnet, daß man in einem geeigneten Nährmedium oder NaCl suspendiert.

12. Verwendung von in vitro-immunrelevant informierten Makrophagen zur Immunisierung von Patienten.

Fig. 1

Fig. 2

Fig. 3

4/4

Fig. 4